**Europäisches Patentamt**

**European Patent Office**

**Office européen des brevets**

(19)

(11) Numéro de publication: **0 132 178**
**B1**

(12)

# FASCICULE DE BREVET EUROPEEN

(45) Date de publication du fascicule du brevet:
**19.11.87**

(21) Numéro de dépôt: **84401303.7**

(22) Date de dépôt: **21.06.84**

(51) Int. Cl.⁴: **A 61 K 37/02**, A 61 K 37/14,
A 61 K 39/395, A 61 K 35/14

```
E R R A T U M
```

(SEITE, SPALTE, ZEILE)
(PAGE, COLUMN, LINE)
(PAGE, COLONNE, LIGNE)

DIE TEXTSTELLE :
TEXT PUBLISHED :
LE PASSAGE SUIVANT :

LAUTET BERICHTIGT:
SHOULD READ :
DEVRAIT ETRE LU :

| DIE TEXTSTELLE | SEITE | SPALTE | ZEILE | LAUTET BERICHTIGT |
|---|---|---|---|---|
| d'un dégré du purification | 2 | 1 | 21/22 | d'un degré de purification |
| revêtues de DEAE Dextrane (voir par exemple le brevet francais no 2 319 399 déposé le 29 juillet 1976. | 2 | | 45 | revêtues de DEAE Dextran (voir par exemple le brevet francais no 2 319 399 déposé le 29 juillet 1976). |
| Merieux < | 2 3 4 | 2 3 | 12 53 45 | > Mérieux |
| Après régénération l'ultrafiltre | 3 | 4 | 28 | Après régénération, l'ultrafiltre |
| une solution d'HCl 0,1N d'alcool | | | 63/64 | une solution d'HCl 0,1N et d'alcool |
| le même module décrite | 4 | 5 | 24/25 | le même module décrit |
| qu'au ph 5,2 | | | 33 | qu'au pH 5,2 |
| bei einem ph-Wert | 5 | 7 | 17 | bei einem pH-Wert |

Tag der Entscheidung über die Berichtigung )
Date of decision on rectification: ) 10.01.88
Date de décision portant sur modification: )

Ausgabe- und Veröffentlichungstag: )
Issue and publication date: ) 16.03.88
Date d'edition et de publication: )

Patbl.Nr)
EPB no:) 88/11
Bull. no:)

Europäisches Patentamt

**European Patent Office**

Office européen des brevets

(19)

(11) Numéro de publication: **0 132 178**
**B1**

(12) **FASCICULE DE BREVET EUROPEEN**

(45) Date de publication du fascicule du brevet:
**19.11.87**

(21) Numéro de dépôt: **84401303.7**

(22) Date de dépôt: **21.06.84**

(51) Int. Cl.⁴: **A 61 K 37/02,** A 61 K 37/14,
A 61 K 39/395, A 61 K 35/14

(54) Procédé de préparation des principales protéines du sang hémolysé sous forme non dénaturée.

(30) Priorité: **07.07.83 FR 8311323**

(43) Date de publication de la demande:
**23.01.85 Bulletin 85/4**

(45) Mention de la délivrance du brevet:
**19.11.87 Bulletin 87/47**

(84) Etats contractants désignés:
**AT BE CH DE GB IT LI LU NL SE**

(56) Documents cités:
**DE - A - 2 638 764**
**FR - A - 2 319 399**
**FR - A - 2 327 256**

(73) Titulaire: **INSTITUT MERIEUX Société anonyme dite:,
17, rue Bourgelat, F-69002 LYON (FR)**

(72) Inventeur: **Tayot, J.L., 11 rue des Greffières, F-69890 La
Tour de Salvagny (FR)**
Inventeur: **Gattel, Paule Annie, 99 route de Strasbourg,
F-69300 Caluire (FR)**
Inventeur: **Tardy, Michel André, 165 bis, rue Joliot Curie,
F-69005 Lyon (FR)**

(74) Mandataire: **Bernasconi, Jean et al, Cabinet Michel
Lemoine 13 Boulevard des Batignolles, F-75008 Paris
(FR)**

**Description**

La présente invention a trait à un procédé de séparation des principales protéines du sang hémolysé afin de permettre l'obtention à l'échelle industrielle, des protéines telles que l'albumine, l'hémoglobine et les gamma-globulines.

Les procédés de fractionnement à l'échelle industrielle des protéines du plasma ou de sérum sanguin reposent sur les techniques de précipitation sélective. L'un des procédés, bien connu, est la méthode de Cohn utilisant la précipitation sélective par l'éthanol. D'autres procédés utilisent d'autres agents chimiques tels que des solvants, le sulfate d'ammonium, l'acide caprylique, le rivanol. Ces différents procédés ont été appliqués principalement à l'obtention et à la purification de l'albumine et des gamma-globulines.

Ils possèdent cependant un certain nombre d'inconvénients, parmi lesquels on peut citer une sélectivité limitée et donc l'obtention d'un degré du purification limité, des pertes en rendement par dénaturation en raison de la formation de polymères ou d'agrégats de protéines pendant les étapes de précipitation, et enfin des opérations industrielles fastidieuses, bruyantes et délicates comme les opérations de centrifugation, nécessitant un personnel habile et compétent.

En outre, dès lors que ces procédés sont mis en œuvre sur du sang hémolysé, par exemple du sang placentaire qui constitue la source la plus importante de protéines sériques, les étapes connues d'élimination de l'hémoglobine conduisent à la dénaturation de celle-ci. Or, il serait souhaitable de disposer de sources importantes d'hémoglobine native en raison notamment de l'intérêt que peut présenter l'hémoglobine, comme source de sang artificiel.

On a donc déjà envisagé de réaliser des étapes de purification de protéines du plasma ou du sang hémolysé par chromatographie, et notamment par chromatographie d'échange d'ions, en utilisant des supports de chromatographie modernes tels que par exemple des microbilles de silice revêtues de DEAE Dextrane (voir par exemple le brevet français no 23 19 399 déposé le 29 juillet 1976.

On a ainsi déjà envisagé de soumettre à une étape de séparation chromatographie le surnageant placentaire d'albumine obtenu après précipitation alcoolique des γ-globulines, à pH 6,8 en présence de 25% d'éthanol, séparant ainsi l'hémoglobine de l'albumine, laquelle était ensuite récupérée par élution à l'aide d'une solution NaCl 1% (voir J. L. Tayot et al.; Chromatography of synthetic and biological polymers, 1978, Vol. 2, pages 95 à 109). Un procédé similaire a également été suggéré dans le brevet français précité dans lequel le surnageant de l'étape de précipitation alcoolique du bloc des globulines est soumis à une séparation chromatographique à un pH compris entre 6 et 7 après dilution dans de l'eau distillée pour diminuer la concentration en alcool, permettant ainsi de séparer l'albumine de l'hémoglobine qui traverse la colonne sans se fixer au support.

Cependant, malgré les avantages que l'on peut attendre de la séparation chromatographique, ces procédés de purification du sang hémolysé n'on pas pu être utilisés à l'échelle industrielle.

Certes, on a déjà prévu de purifier l'albumine d'origine placentaire par des étapes de séparation chromatographique mais à condition d'éliminer tout d'abord l'essentiel de l'hémoglobine par une précipitation dénaturante, par exemple au chloroforme (voir J. L. Tayot et al.; Coopération internationale et dérivés sanguins-Talloires 1981; Ed. fondation Merieux), mais un tel procédé conduit à la dénaturation irréversible de l'hémoglobine. Par contre les opérations de séparation chromatographique précitées du surnageant d'origine placentaire contenant l'albumine et l'hémoglobine n'ont pas pu atteindre le stade industriel, notamment en raison d'une légère fixation d'hémoglobine sur le support, difficile à éluer complètement de sorte que l'accumulation des pigments, de cycle en cycle, sur le support entrave la capacité de fixation et la durée d'utilisation. De plus la solution injectée dans la colonne est très difficile à clarifier. Elle se trouble en continu, ce qui en rend la filtration très difficile. En outre elle contient des sels qui diminuent fortement la fixation de l'albumine.

L'invention se propose, en conséquence, de remédier à ces inconvénients et de fournir un procédé de séparation des principales protéines du sang hémolysé, sous forme non dénaturée, suceptible de séparer, avec un degré de pureté extrêmement élevé, les protéines principales telles que albumine, hémoglobine, et si on le désire, γ-globulines.

Un autre objectif de l'invention est de fournir un tel procédé qui permette de traiter de façon économique des quantités extrêmement importantes de sang hémolysé, telles que par exemple plusieurs tonnes ou plusieurs dizaines de tonnes de placenta par jour correspondant à plusieurs dizaines de milliers de litres de sang hémolysé.

L'invention a pour objet un procédé de séparation des principales protéines du sang hémolysé, et notamment de l'albumine et de l'hémoglobine, sous forme non dénaturée, dans lequel on effectue une étape de séparation chromatographique de l'albumine et de l'hémoglobine, caractérisé en ce que l'on soumet un sang hémolysé à une étape de clarification, puis, de préférence avant toute précipitation alcoolique de γ-globulines, on effectue une chromatographie du sang hémolysé sur un support chromatographique échangeur d'anions à un pH supérieur à 4,8 et inférieur à 6,8, de préférence inférieur à 6 dans le cas d'une précipitation alcoolique préalable des γ-globulines, le pH étant de préférence compris entre 5 et 6, après quoi on récupère séparément l'hémoglobine sortant de la colonne de chromatographie et l'albumine obtenue après élution.

De façon particulièrement préférée, on réalise la clarification à un pH compris entre 4 et 6, de préférence entre 4,8 et 5,4, en présence d'alcool, de préférence éthanol, à une concentration inférieure à 15% et de préférence voisine de 8%.

Le sang hémolysé étant en général dilué, on réalise une étape de concentration, conduite sur des systèmes d'ultrafiltration ayant un seuil de coupure de préférence de l'ordre de 10000 daltons, de préférence de façon à concentrer au moins quatre fois le sang hémolysé, ainsi qu'une diafiltration.

Dans le cas, qui n'est pas préféré, dans lequel le sang hémolysé a subi préalablement des étapes de précipitation alcoolique, notamment pour la séparation des γ-globulines, il est nécessaire d'effectuer une dilution dans de l'eau distillée ou un liquide analogue de façon que le taux d'alcool ne dépasse pas 15%. Ceci entraîne la nécessité de travailler sur des volumes très importants et présente d'autre part des inconvénients dus au fait que les additifs nécessaires dans le cas de la précipitation alcoolique des γ-globulines diminuent les possibilités d'adsorption du support chromatographique.

Si l'on souhaite séparer les globulines et notamment γ-globulines de l'hémoglobine purifiée, débarrassée de l'albumine, on peut avantageusement, après avoir obtenu cette hémoglobine par l'étape de chromatographie, traiter le filtrat par addition d'éthanol à environ 25%, à froid, avec ajustement du pH vers 7. Le précipité d'immunoglobulines peut alors être récupéré par exemple par centrifugation ou filtration et subir ensuite les opérations de purification éventuellement nécessaires. Le surnageant ou filtrat correspondant contient l'hémoglobine sous forme native pratiquement pure. De préférence, cette hémoglobine est diluée avec au moins un volume d'eau distillée de façon à abaisser la concentration en éthanol suffisamment pour éviter toute dénaturation ultérieure. La solution peut être soumise à une opération d'ultrafiltration et de diafiltration pour récupérer l'hémoglobine native concentrée prête à subir d'autres opérations de purification ou autres traitements nécessaires à sa transformation en substitut artificiel du sang.

Conformément à l'invention, le support de chromatographie est un support à très hautes propriétés mécaniques, comprenant par exemple un substrat en billes de silice.

Le support de chromatographie est de préférence le support dénommé Sphérodex® et décrit par exemple dans le brevet français précité ou dans le brevet luxembourgeois no 73094 déposé le 29 juillet 1975, constitué d'une silice poreuse revêtue de DEAE Dextran et préparée à l'Institut Merieux.

D'autres avantages et caractéristiques de l'invention apparaîtront à la lecture de la description suivante, faite à titre d'exemple non limitatif.

1) Etape de clarification du sang placentaire hémolysé

150 kg de placentas humains sont broyés à l'état congelé et dispersés dans 150 litres d'eau, additionnés de 22 litres d'éthanol pour atteindre une température de 0 °C et une concentration alcoolique d'environ 8%. La suspension ainsi obtenue est agitée vigoureusement puis soumise à une filtration sur pressoir de volume 400 litres équipé d'une toile de porosité 50 microns, pour séparer le jus sanguin des tissus placentaires. Le liquide récupéré occupe un volume de 270 litres. Il est additionné d'acide acétique ou d'acide chlorhydrique N indifféremment, pour ajuster le pH à 5,10 sous agitation à 0 °C. Après 2 heures d'attente, la suspension est injectée à 300 l/h sur centrifugeuse. Un précipité voisin de 7 à 10 kg suivant les jours est éliminé. Le sang obtenu dans le surnageant est parfaitement limpide et occupe un volume de 260 litres environ.

2) Etape de concentration et diafiltration du sang clarifié

Un ultrafiltre équipé d'une membrane de seuil de coupure 10000 daltons est utilisé. Par exemple deux cartouches spirales de 5 m² chacune, commercialisées par Millipore, conviennent bien pour cette opération. Après concentration à environ 60 litres, le pH est ajusté à 5,50 avec de la soude N et on diafiltre ensuite à volume constant avec environ 200 litres d'eau déminéralisée. Le débit d'élimination de l'ultrafiltrat est d'environ 50 l/h pour une pression de 4 bars à l'entrée. L'opération totale dure environ 8 heures à +4 °C. Le pH final est voisin de 5,25 et n'est pas modifié. Après régénération l'ultrafiltre peut être ainsi réutilisé plus de 200 fois sans colmatage.

Après repos de 15 heures à +4 °C, le sang concentré contient un léger précipité d'euglobulines qui est éliminé par centrifugation. Le culot obtenu représente un poids de 100 à 300 grammes selon les lots. Le sang concentré clarifié d'un volume de 120 litres est ensuite filtré sans problèmes sur une membrane de porosité 0,2 μ et conservé stérilement dans l'attente de l'étape suivante.

3) Etape de séparation chromatographique

Une colonne de diamètre 16 cm et de hauteur 1 m est remplie de 9 kg de Sphérodex®. Elle est équilibrée en tampon phosphate 0,01 M pH 5,25, dans des conditions stériles déjà décrites (Tayot et coll.: Coopération Internationale et dérivés sanguins, 1981, Editions Fondation Merieux, Lyon). Les 120 litres de sang concentré et filtré obtenus précédemment sont injectés dans la colonne à un débit de 40 l/h, de manière stérile à travers un filtre de porosité 0,2 μ. La colonne est ensuite rincée par 40 litres de tampon PO₄ 0,01 M pH 5,25. Le filtrat d'un volume de 135 litres, contenant l'hémoglobine et les immunoglobulines, est totalement débarrassé de l'albumine. Il est conservé à +2 °C en attendant l'étape suivante. L'albumine fixée sur la colonne avec d'autres protéines est ensuite éluée par injection de 60 litres d'une solution de NaCl 20 g/l. Un volume de 50 litres suffit pour récupérer quantitativement l'albumine, soit environ 1200 g (8 g/kg placentas en moyenne). Cette solution d'albumine doit ensuite être soumise à d'autres étapes connues de purification pour être utilisable en thérapeutique humaine.

La colonne après lavage par une solution d'HCl 0,1 N d'alcool est ensuite prête pour un nouveau cycle d'utilisation.

Plus de 50 cycles ont été effectués sur la même colonne, sans perte d'efficacité.

**4) Séparation finale des gamma-globulines et de l'hémoglobine**

Le filtrat précédent (135 litres) est ajusté en chlorure de sodium 8 g/l, ajusté à pH 7, refroidi à 0 °C. 45 litres d'éthanol refroidi à −20 °C sont ajoutés progressivement, sous agitation et la température est ajustée à −5 °C en final. Après un repos d'une nuit, la suspension obtenue est centrifugée à un débit de 300 l/h.

Le précipité de gammaglobulines obtenu pèse 900 grammes en moyenne sur plusieurs lots, et doit être ensuite soumis à d'autres opérations de purification déjà connues, pour préparer des immunoglobulines utilisables en thérapeutique humaine.

Le surnageant obtenu est parfaitement limpide. Il contient l'hémoglobine pratiquement débarrassée d'immunoglobulines. Il est dilué avec 300 litres d'eau à 0 °C pour faire descendre la concentration alcoolique en dessous de 10%. Cette solution est concentrée par ultrafiltration sur le même module décrite précédemment et peut être diafiltrée en final pour ajuster la force ionique et la composition de la solution en fonction de son utilisation future. La quantité d'hémoglobine ainsi obtenue est de 3700 g, soit un rendement voisin de 25 g/kg placentas.

Des essais effectués à un pH de 5,0 montrent une diminution de la capacité de la colonne à retenir l'albumine alors qu'au ph 5,2 l'albumine est totalement retenue. Des essais effectués à pH 6,5 ont montré que si l'albumine est entièrement retenue, une contamination du support par les pigments de l'hémoglobine commence à apparaître.

Dans le cas, non préféré, dans lequel on soumet le sang hémolysé à une précipitation alcoolique à pH 7 pour séparer les γ-globulines, l'étape de clarification à pH 5,1 et 8% d'alcool est conduite avant et/ou après la précipitation de la fraction globulinique.

**Revendications**

1. Procédé de séparation des principales protéines du sang hémolysé, et notamment de l'albumine et de l'hémoglobine, sous forme non dénaturée, dans lequel on effectue une étape de séparation chromatographique de l'albumine et de l'hémoglobine, caractérisé en ce que l'on soumet un sang hémolysé à une étape de clarification, puis, on effectue une chromatographie du sang hémolysé sur un support chromatographique échangeur d'anions à un pH supérieur à 4,8 et inférieur à 6,8, après quoi on récupère séparément l'hémoglobine sortant de la colonne de chromatographie et l'albumine obtenue après élution.

2. Procédé selon la revendication 1, caractérisé en ce que l'on effectue la chromatographie à un pH compris entre 5 et 6.

3. Procédé selon l'une des revendications 1 et 2, caractérisé en ce que l'on effectue l'étape de clarification à un pH compris entre 4 et 6 en présence d'alcool à une concentration inférieure à 15%.

4. Procédé selon la revendication 3, caractérisé en ce que l'on réalise la clarification à un pH compris entre 4,8 et 5,4.

5. Procédé selon l'une des revendications 3 et 4, caractérisé en ce que l'on réalise l'étape de clarification en présence d'alcool à une concentration voisine de 8%.

6. Procédé selon l'une quelconque des revendications 3 à 5, caractérisé en ce que l'on réalise l'étape de clarification en présence d'éthanol.

7. Procédé selon l'une quelconque des revendications 1 à 6, caractérisé en ce que l'on effectue la chromatographie avant toute séparation des γ-globulines.

8. Procédé selon la revendication 7, caractérisé en ce que l'on sépare les γ-globulines de l'hémoglobine purifiée.

9. Procédé selon la revendication 8, caractérisé en ce que l'on sépare les γ-globulines par précipitation à l'alcool à 25% environ, à un pH voisin de 7.

10. Procédé selon l'une quelconque des revendications 1 à 9, caractérisé en ce que l'on utilise pour la chromatographie un support de silice poreuse revêtue de DEAE dextran.

11. Procédé selon l'une quelconque des revendications 1 à 10, caractérisé en ce que l'on équilibre la colonne de chromatographie en tampon phosphate 0,01 M puis qu'on la rince dans le même tampon, l'albumine étant éluée par une solution de NaCl 20 g/l.

12. Procédé selon l'une quelconque des revendications 1 à 11, caractérisé en ce qu'avant la chromatographie, on effectue une concentration d'un sang hémolysé dilué, notamment par ultrafiltration et diafiltration.

**Patentansprüche**

1. Verfahren zum Abtrennen der wichtigsten Proteine des hämolysierten Blutes, und insbesondere des Albumins und des Hämoglobins, in einer nichtdenaturierten Form, bei dem man einen chromatographischen Abtrennungsschritt des Albumins und des Hämoglobins durchführt, dadurch gekennzeichnet, dass man hämolysiertes Blut einem Klärschritt unterwirft, dass man dann eine Chromatographie des hämolysierten Blutes auf einem chromatographischen Anionenaustauschträger bei einem pH-Wert oberhalb 4,8 und unterhalb 6,8 durchführt, worauf man gesondert das aus der Chromatographiekolonne austretende Hämoglobin und das nach Elution erhaltene Albumin gewinnt.

2. Verfahren nach Anspruch 1, dadurch gekennzeichnet, dass man die Chromatographie bei einem pH-Wert zwischen 5 und 6 ausführt.

3. Verfahren nach Anspruch 1 oder 2, dadurch gekennzeichnet, dass man den Klärschritt bei einem pH-Wert zwischen 4 und 6 in Gegenwart von Alkohol einer Konzentration unter 15% ausführt.

4. Verfahren nach Anspruch 3, dadurch gekennzeichnet, dass man die Klärung bei einem pH-Wert zwischen 4,8 und 5,4 vornimmt.

5. Verfahren nach Anspruch 3 oder 4, dadurch gekennzeichnet, dass man den Klärschritt in Gegenwart von Alkohol einer Konzentration nahe 8% vornimmt.

6. Verfahren nach einem der Ansprüche 3 bis 5, dadurch gekennzeichnet, dass man den Klärschritt in Gegenwart von Äthanol vornimmt.

7. Verfahren nach einem der Ansprüche 1 bis 6, dadurch gekennzeichnet, dass man die Chromatographie vor jeglicher Abtrennung der γ-Globuline durchführt.

8. Verfahren nach Anspruch 7, dadurch gekennzeichnet, dass man die γ-Globuline des gereinigten Hämoglobins abtrennt.

9. Verfahren nach Anspruch 8, dadurch gekennzeichnet, dass man die γ-Globuline durch Ausfällen mit etwa 25%igem Alkohol, bei einem ph-Wert nahe 7 abtrennt.

10. Verfahren nach einem der Ansprüche 1 bis 9, dadurch gekennzeichnet, dass man für die Chromatographie einen porösen Siliziumträger verwendet, der mit DEAE dextran beschichtet ist.

11. Verfahren nach einem der Ansprüche 1 bis 10, dadurch gekennzeichnet, dass man die Chromatographiekolonne mit einem Phosphatpuffer von 0,01 M neutralisiert und dass man sie dann in demselben Puffer wäscht, wobei das Albumin durch eine NaCl-Lösung von 20 g/l eluiert wird.

12. Verfahren nach einem der Ansprüche 1 bis 11, dadurch gekennzeichnet, dass man vor der Chromatographie verdünntes hämolysiertes Blut konzentriert, insbesondere durch Ultrafiltration und Diafiltration.

## Claims

1. A process for separating the principal proteins from haemolyzed blood, and in particular the albumin and the haemoglobin in the non-denatured form, in which a chromatographic separation of the albumin and the haemoglobin is effected, characterized in that a haemolyzed blood is subjected to a clarification step, then a chromatography of the haemolyzed blood is effected on an anion exchanging chromatographic support at a pH higher than 4.8 and lower than 6.8, after which the haemoglobin issuing from the chromatographic column and the albumin obtained after elution are recovered separately.

2. A process according to claim 1, characterized in that the chromatography is effected at a pH of between 5 and 6.

3. A process according to claim 1 or 2, characterized in that the clarification step is effected at a pH between 4 and 6 in the presence of alcohol at a concentration lower than 15%.

4. A process according to claim 3, characterized in that the clarification is effected at a pH of between 4.8 and 5.4.

5. A process according to claim 3 or 4, characterized in that the clarification step is effected in the presence of alcohol at a concentration of around 8%.

6. A process according to any one of claims 3 to 5, characterized in that the clarification step is effected in the presence of ethanol.

7. A process according to any one of claims 1 to 6, characterized in that the chromatography is effected before any separation of the γ globulins.

8. A process according to claim 7, characterized in that the γ globulins are separated from the purified haemoglobin.

9. A process according to claim 8, characterized in that the γ globulins are separated by precipitation with alcohol at about 25% and with a pH in the region of 7.

10. A process according to any one of claims 1 to 9, characterized in that a support of porous silica coated with DEAE dextran is employed for the chromatography.

11. A process according to any one of claims 1 to 10, characterized in that the chromatographic column is equilibrated with an 0.01 M phosphate buffer, then is rinsed in the same buffer, the albumin being eluted with a 20 g/l solution of NaCl.

12. A process according to any one of claims 1 to 11, characterized in that a concentration of a diluted haemolyzed blood, in particular by ultrafiltration and diafiltration, is effected before the chromatography.